# EUROPEAN PATENT SPECIFICATION

(11) **EP 3 369 385 B1**
(45) Date of publication and mention of the grant of the patent: **20.01.2021**
(21) Application number: 18159314.6
(22) Date of filing: 28.02.2018
(51) Int. Cl.: A61B 17/072, A61B 17/00, A61B 90/00

(54) **POWERED SURGICAL DEVICES HAVING TISSUE SENSING FUNCTION**
ANGETRIEBENE CHIRURGISCHE VORRICHTUNGEN MIT GEWEBEERFASSUNGSFUNKTION
DISPOSITIFS CHIRURGICAUX ÉLECTRIQUES AYANT UNE FONCTION DE DÉTECTION DE TISSU

(30) Priority: 03.03.2017 US 201762466481 P; 02.02.2018 US 201815887024
(43) Date of publication of application: 05.09.2018
(73) Proprietor: Covidien LP, Mansfield, MA 02048 (US)
(72) Inventor: ESCHBACH, Matthew, Cheshire, Connecticut 06410 (US)
(74) Representative: Soames, Candida Jane

(56) References cited:
- EP-A1- 2 926 743
- EP-A2- 1 728 475
- EP-A2- 2 992 839
- US-A1- 2015 122 870
- US-A1- 2016 100 839

## Description

### TECHNICAL FIELD

The present disclosure relates generally to surgical devices. More particularly, the present disclosure relates to end effectors for powered handheld electromechanical instruments including sensors for in vivo monitoring of tissue behavior in real-time during surgical procedures.

### BACKGROUND

One type of surgical device is a linear clamping, cutting and stapling device. Such a device may be employed in a surgical procedure to resect a cancerous or anomalous tissue from a gastro-intestinal tract. Conventional linear clamping, cutting and stapling instruments include a pistol grip-styled structure having an elongated shaft and distal portion. The distal portion includes a pair of scissors-styled gripping elements, which clamp tissue, such as the open ends of the colon, closed. In this device, one of the two scissors-styled gripping elements, such as an anvil portion, moves or pivots relative to the overall structure, whereas the other gripping element remains fixed relative to the overall structure. The actuation of this scissoring device (the pivoting of the anvil portion) is controlled by a grip trigger maintained in the handle.

In addition to the scissoring device, the distal portion also includes a stapling mechanism. The fixed gripping element of the scissoring mechanism includes a staple cartridge receiving region and a mechanism for driving the staples up through the clamped end of the tissue against the anvil portion, thereby sealing the previously opened end. The scissoring elements may be integrally formed with the shaft or may be detachable such that various scissoring and stapling elements may be interchangeable.

A number of surgical device manufacturers have developed product lines with proprietary powered drive systems for operating and/or manipulating the surgical device. In many instances the surgical devices include a powered handle assembly, which is reusable, and a disposable end effector or the like that is selectively connected to the powered handle assembly prior to use and then disconnected from the end effector following use in order to be disposed of, or in some instances sterilized for re-use.

The use of powered electro and endomechanical surgical staplers, including intelligent battery power, has grown tremendously over the past few decades. Advanced technology and informatics within these intelligent battery-powered stapling devices provide the ability to gather clinical data and drive design improvements to ultimately improve patient outcomes.

For example, some powered stapling devices use current sensors to detect electrical current draw from a motor of the device, or a strain gauge along a drive assembly of the device, as an indicator of the forces required to compress tissue, form staples, and transect the tissue. Data collected from these sensors are used to control the speed of firing, which has been shown to improve staple formation by slowing the stapler speed and lowering the firing force. The data may also be used in other aspects of the stapling process, such as detecting end stop and emergency stopping to prevent damage to the end effector. These powered stapling devices, however, are limited in obtaining data regarding the in vivo mechanical behavior (e.g., the mechanical force, stress, and strain) of the tissue. Without this data, it is difficult to prevent acute trauma due to stress thresholds that were exceeded in the tissue while performing a stapling procedure.

European Patent Application EP 2 926 743 A1 discloses a surgical instrument comprising a test end effector including a test jaw assembly having a pair of jaws configured to clamp about tissue and at least one sensor configured to measure at least one tissue property; and a handle assembly configured to couple to the treatment end effector. The handle assembly includes: a drive assembly; a motor operatively coupled to the drive assembly; and a controller operatively coupled to the motor, the controller configured to control operation of the motor to actuate the test end effector to measure the at least one tissue property and to determine, based on the at least one tissue property, at least one suitable treatment end effector.

United States Patent Application US 2015/0122870 A1 discloses a surgical device comprising an instrument and and end effector, wherein the end effector includes a first jaw and a second jaw and an identifier at a proximal end of the end effector. The identifier is configured to transmit sensor data to a microcontroller of the instrument. The first and second jaw members include strain gauges to directly measure the load being exerted thereon. These measurements are then transmitted to the microcontroller of the instrument which analyses the angle and/or strain measurements and alerts the user of the stress on the end effector.

European Patent Application EP 2 992 839 A2 describes surgical staplers including a staple cartridge having one or more light-emitting diodes positioned at the edges of a tissue contacting surface thereof.

United States Patent Application US 2016/0100839 A1 describes a multi-fire surgical stapler having an electronic counter, a lockout, and a visual indicator.

### SUMMARY

The present invention provides an end effector for a surgical device, the end effector comprising: a body portion; and a tool assembly disposed at a distal end of the body portion, the tool assembly including: an anvil assembly having a tissue contacting surface and an outer surface; a cartridge assembly pivotally coupled to the anvil assembly, the cartridge assembly having a tissue contacting surface and an outer surface and a sensor disposed on the outer surface of the anvil assembly or on the outer surface of the cartridge assembly, the sensor configured to measure a mechanical property of at least one of the anvil assembly or the cartridge assembly, at least during an approximation of the anvil assembly and the cartridge assembly onto the target tissue; the end effector further comprises: a microcontroller disposed within the body portion, the microcontroller electrically coupled to the sensor and configured to receive sensor data of the sensor corresponding to the mechanical property of at least one of the anvil assembly or the cartridge assembly and configured to process the sensor data into a property of the target tissue, and wherein the microcontroller is configured to control a function of the tool assembly in response to the processed sensor data; and a memory disposed within the body portion, the memory coupled to the microcontroller and configured to store at least one of the sensor data or the processed sensor data.

Suitably, the sensor is a pressure sensor. In embodiments, the sensor is a strain gauge.

Suitably, the sensor is one of a plurality of sensors disposed on the outer surface of the anvil assembly or the cartridge assembly. In these embodiment, suitably at least two of the plurality of sensors are configured to measure the same mechanical property. More suitably, the microcontroller is configured to process the mechanical property into target tissue stress.

Suitably, the microcontroller is configured to continuously monitor the sensor to collect the sensor data.

### BRIEF DESCRIPTION OF THE DRAWINGS

Embodiments of the present disclosure are described herein with reference to the accompanying drawings, wherein:
FIG. 1 is a perspective view of a surgical device including a handle housing, an adapter assembly, and an end effector in accordance with an embodiment of the present disclosure;
FIG. 2A is a perspective view, with parts separated, of the handle housing of the surgical device of FIG. 1;
FIG. 2B is a perspective view, with parts separated, of a motor assembly and a control assembly of the handle housing of FIGS. 1 and 2A;
FIG. 3A is a perspective view of the adapter assembly of the surgical device of FIG. 1;
FIG. 3B is a perspective view of an electrical assembly of the adapter assembly of FIGS. 1 and 3A;
FIG. 3C is a cutaway view of a distal portion of the adapter assembly of FIGS. 1 and 3A;
FIG. 3D is a perspective view of an annular member and a switch of the adapter assembly of FIGS. 1, 3A, and 3C;
FIG. 4A is a perspective view of the end effector of the surgical device of FIG. 1;
FIG. 4B is a perspective view of an anvil assembly and a drive assembly of the end effector of FIGS. 1 and 4A;
FIG. 4C is a perspective view of an inner housing of the end effector of FIGS. 1 and 4A; and
FIG. 4D is a perspective view, with parts separated, of the end effector of the surgical device of FIGS. 1 and 4A.

### DETAILED DESCRIPTION OF EMBODIMENTS

As described above, the end effector of the present invention comprises a microcontroller disposed within the body portion and configured to process the sensor data into a property of the target tissue and to control a function of the tool assembly in response to the processed sensor data. Embodiments of surgical devices comprising a microcontroller disposed within the handle assembly and, at the same time, not comprising a microcontroller disposed within the body portion of the end effector as claimed, do not form part of the present invention.

Embodiments of the present disclosure are now described in detail with reference to the drawings in which like reference numerals designate identical or corresponding elements in each of the several views. Throughout this description, the term "proximal" refers to a portion of a device, or component thereof, that is closer to a user, and the term "distal" refers to a portion of the device, or component thereof, that is farther from the user.

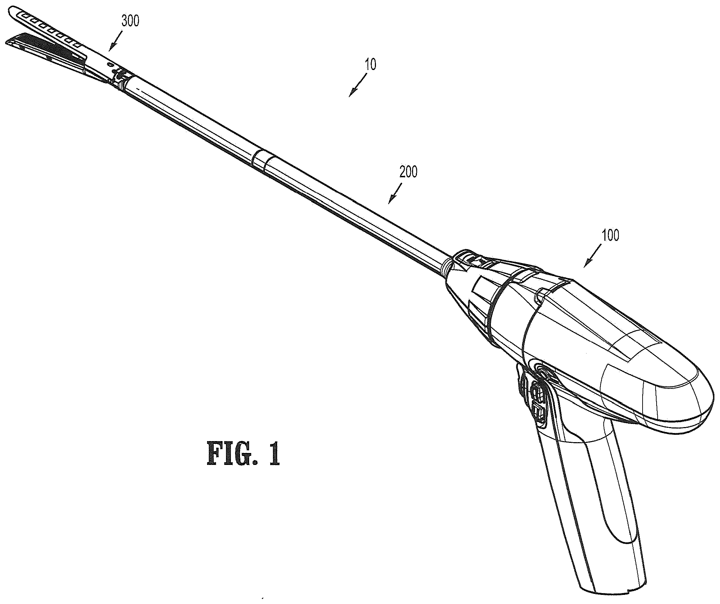

Turning now to FIG. 1, a surgical device 10, in accordance with an embodiment of the present disclosure, is in the form of a powered handheld electromechanical instrument. The surgical device 10 includes a handle assembly 100, an adapter assembly 200, and a tool assembly or end effector 300. The handle assembly 100 is configured for selective connection with the adapter assembly 200 and, in turn, the adapter assembly 200 is configured for selective connection with the end effector 300.

The handle assembly 100, the adapter assembly 200, and the end effector 300 will only further be described to the extent necessary to disclose aspects of the present disclosure. For a detailed description of the structure and function of exemplary handle and adapter assemblies, and end effectors, reference may be made to commonly owned U.S. Patent Publication No. 2016-0310134 ("the '134 Publication").

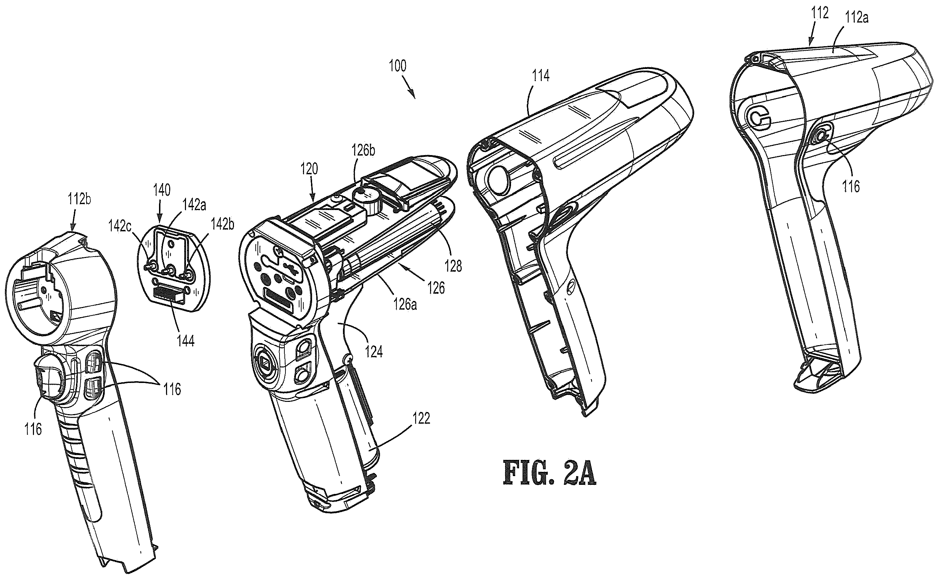

With reference now to FIG. 2A, the handle assembly 100 includes an outer housing shell 112, including a proximal half-section 112a and a distal half-section 112b, and an inner handle housing 114 disposed within the outer housing shell 112. The outer housing shell 112 includes a plurality of actuators 116 (e.g., finger-actuated control buttons, knobs, toggles, slides, interfaces, and the like) for activating various functions of the surgical device 10 (FIG. 1), and the inner handle housing 114 houses a power-pack 120 configured to power and control various operations of the surgical device 10.

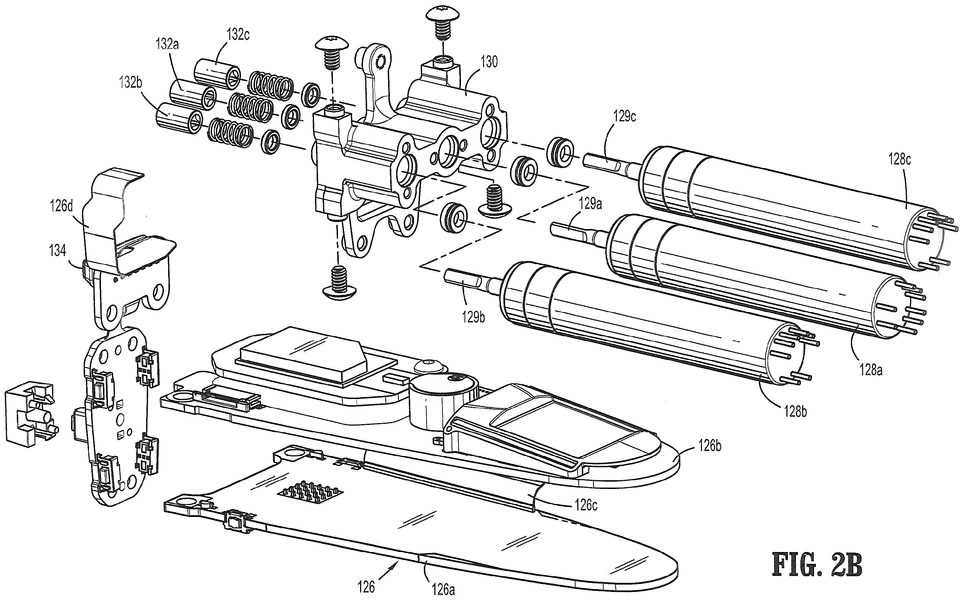

As shown in FIGS. 2A and 2B, the power-pack 120 includes a rechargeable battery 122 configured to supply power to any of the electrical components of the surgical device 10, a battery circuit board 124, and a controller circuit board 126. The controller circuit board 126 includes a motor controller circuit board 126a, a main controller circuit board 126b, and a first ribbon cable 126c interconnecting the motor controller circuit board 126a and the main controller circuit board 126b. The motor controller circuit board 126a is communicatively coupled with the battery circuit board 124 enabling communication therebetween and between the battery circuit board 124 and the main controller circuit board 126b.

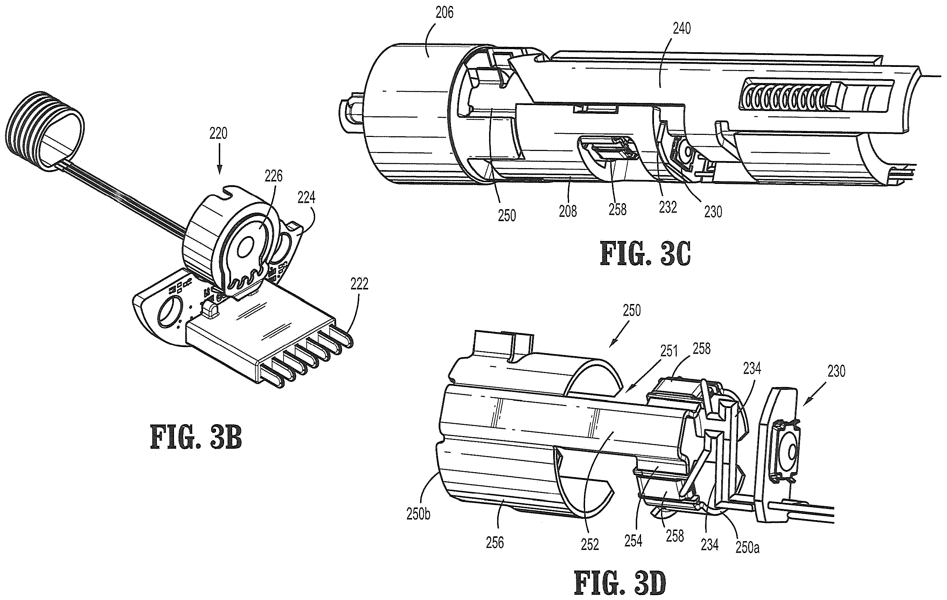

The main controller circuit board 126a includes a 1-wire communication system including three 1-wire buses which enables communication between the power-pack 120 and the battery 122, the power-pack 120 and the adapter assembly 200 (FIG. 1), and the power-pack 120 and the outer shell housing 112. Specifically, with regard to communication between the power-pack 120 and the adapter assembly 200, the 1-wire bus establishes a communication line between a 1-wire master chip of the main controller circuit board 126b and a 1-wire memory chip of a circuit board 224 (FIG. 3B) of the adapter assembly 200. This communication line allows for calibration and communication of data and control signals between the handle assembly 100 and the adapter assembly 200, and enables information stored in the 1-wire memory chip of the circuit board 224 of the adapter assembly 200 to be accessed, updated, and/or incremented by the power-pack 120.

The power-pack 120 further includes motors 128 (e.g., a first motor 128a, a second motor 128b, and a third motor 128c) each electrically connected to the controller circuit board 126 and the battery 122. The motors 128a, 128b, 128c are disposed between the motor controller circuit board 126a and the main controller circuit board 126b. Each of the motors 128a, 128b, 128c includes a respective motor shaft 129a, 129b, 129c extending therefrom for transmitting rotative forces or torque.

Each of the motors 128a, 128b, 128c is controlled by a respective motor controller (not shown) disposed on the motor controller circuit board 126a, and each motor controller is electrically coupled to a main controller or master chip disposed on the main controller circuit board 126b via the first ribbon cable 126c which connects the motor controller circuit board 126a with the main controller circuit board 126b. The master chip is also coupled to memory, which is also disposed on the main controller circuit board 126b.

Each of the motor 128a, 128b, 128c is supported on a motor bracket 130 such that the motor shafts 129a, 129b, 129c are rotatably disposed within respective apertures of the motor bracket 130. The motor bracket 130 rotatably supports three rotatable drive connector sleeves 132a, 132b, 132c that are keyed to respective motor shafts 129a, 129b, 129c of the motors 128a, 128b, 128c. The drive connector sleeves 132a, 132b, 132c non-rotatably receive proximal ends of respective coupling shafts 142a, 142b, 142c of a plate assembly 140 of the handle assembly 100, when the power-pack 120 is disposed within the outer shell housing 112.

The motor bracket 130 also supports an electrical adapter interface receptacle 134. The electrical adapter interface receptacle 134 is in electrical connection with the main controller circuit board 126b by a second ribbon cable 126d. The electrical adapter interface receptacle 134 defines a plurality of electrical slots for receiving respective electrical contacts or blades extending from a pass-through connector 144 of the plate assembly 140 of the handle assembly 100.

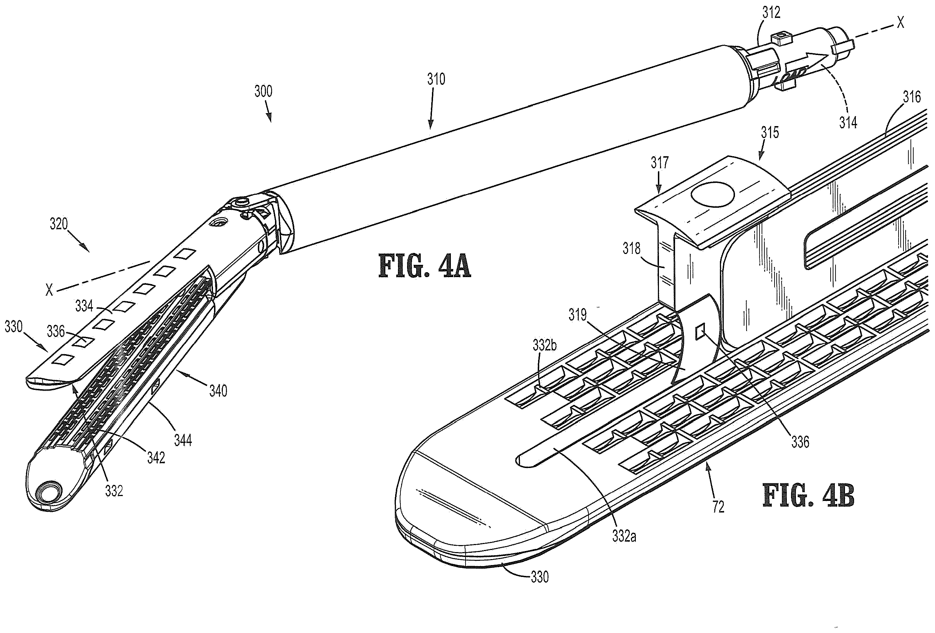

Rotation of the motor shafts 129a, 129b, 129c by respective motors 128a, 128b, 128c function to drive shafts and/or gear components of the adapter assembly 200 in order to perform the various operations of the surgical device 10. In particular, the motors 128a, 128b, 128c of the power-pack 120 are configured to drive shafts and/or gear components of the adapter assembly 200 in order to selectively move a tool assembly 320 (FIG. 4A) of the end effector 300 relative to a proximal body portion 310 (FIG. 4A) of the end effector 300, to rotate the end effector 300 about a longitudinal axis "X" (FIG. 4A), to move a cartridge assembly 340 (FIG. 4A) relative to an anvil assembly 330 (FIG. 4A) of the end effector 300, and/or to fire staples from within the cartridge assembly 340 of the end effector 300.

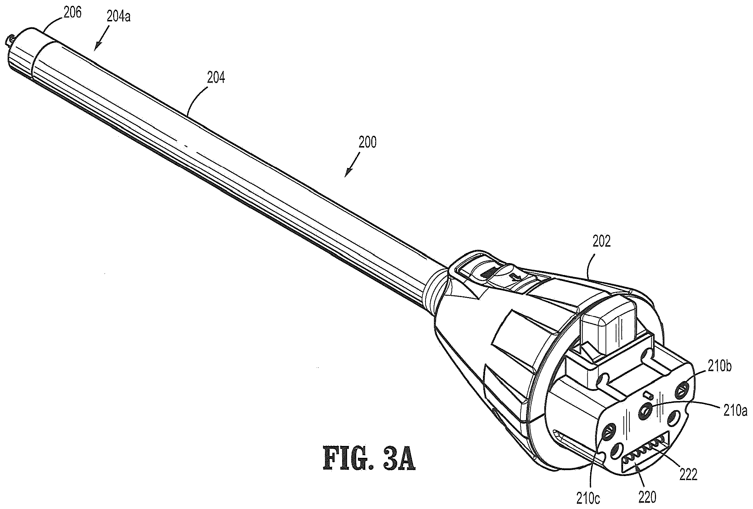

Referring now to FIG. 3A, the adapter assembly 200 includes an outer knob housing or connector housing 202 and an outer tube or sleeve 204 extending distally from the outer knob housing 202 and terminating at a distal cap 206. The outer knob housing 202 is configured for operable connection to the handle assembly 100 (FIG. 1) and the outer tube 204 is configured for operable connection to the end effector 300 (FIG. 1).

Rotatable connector sleeves 210a, 210b, 210c are disposed within the outer knob housing 202 and are configured and adapted to mate, through a keyed and/or substantially non-rotatable interface, with respective coupling shafts 142a, 142b, 142c (FIG. 2A) of the plate assembly 140 of the handle housing 100 such that rotation of each of the coupling shafts 142a, 142b, 142c causes a corresponding rotation of the corresponding connector sleeve 210a, 210b, 210c of the adapter assembly 200. The mating of the coupling shafts 142a, 142b, 142c of the handle assembly 100 with the connector sleeves 210a, 210b, 210c of the adapter assembly 200 allows rotational forces to be independently transmitted via each of the three respective connector interfaces. The coupling shafts 142a, 142b, 142c of the handle assembly 100 are configured to be independently rotated by respective motors 128a, 128b, 128c such that rotational force(s) are selectively transferred from the motor(s) 128a, 128b, 128c of the handle assembly 100 to the adapter assembly 200.

Adapter assembly 200 includes a plurality of force/rotation transmitting/converting assemblies (not shown), each disposed within an inner housing assembly (not shown) of the outer knob housing 202 and the outer tube 204. Each force/rotation transmitting/converting assembly is configured and adapted to transmit/convert a speed/force of rotation (e.g., increase or decrease) of the coupling shafts 142a, 142b, 142c (FIG. 2A) of the handle assembly 100 before transmission of such rotational speed/force to the end effector 300.

Specifically, each force/rotation transmitting/converting assembly is configured and adapted to transmit or convert a rotation of the first, second and third coupling shafts 142a, 142b, 142c of the handle assembly 100 into: axial translation of an articulation bar (not shown) of the adapter assembly 200 to effectuate articulation of the end effector 300 (FIG. 1); a rotation of a ring gear (not shown) of the adapter assembly 200 to effectuate rotation of the adapter assembly 200, and thus, the end effector 300; or axial translation of a distal drive member (not shown) of the adapter assembly 200 to effectuate closing, opening, and firing of the end effector 300.

As shown in FIG. 3B, in conjunction with FIG. 3A, an electrical assembly 220 is supported on and in outer knob housing 202. The electrical assembly 220 includes a plurality of electrical contact blades 222 supported on a circuit board 224 for electrical connection to pass-through connector 144 (FIG. 2A) of the plate assembly 140 of the handle assembly 100. The electrical assembly 220 also includes a strain gauge 226 electrically connected to the circuit board 224 for closed-loop feedback of firing/clamping loads exhibited by the adapter assembly 200 and regulated by the power-pack 120 (FIG. 2A), which sets the speed current limit on the appropriate motor 128a, 128b, 128c (FIG. 2B).

The circuit board 224 includes a memory configured to store data relating to the adapter assembly 200 such as unique ID information (electronic serial number); type information; status information; whether an end effector has been detected, identified, and verified; usage count data; and assumed autoclave count data. The electrical assembly 220 serves to allow for calibration and communication of information (e.g., identifying information, life-cycle information, system information, force information) to the main controller circuit board 126b (FIG. 2A) of the power-pack 120 via the electrical adapter interface receptacle 134 (FIG. 2B) of the power-pack 120 of the handle assembly 100.

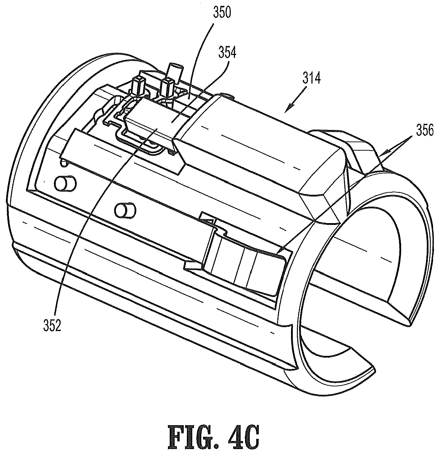

With reference now to FIG. 3C, in conjunction with FIG. 3A, the adapter assembly 200 further includes a switch 230, a sensor link or switch actuator 240, and an annular member 250, each of which is disposed within a distal portion 204a of the outer tube 204. The switch 230 is configured to toggle in response to a coupling of the end effector 300 (FIG. 1) to the outer tube 204. The switch 230 is mounted on a printed circuit board 232 that is electrically connected with the controller circuit board 126 (FIG. 2A) of the power-pack 120 of the handle housing 100. The switch 230 is configured to couple to a memory 352 (FIG. 4C) of the end effector 300. The memory 352 of the end effector 300 is configured to store data pertaining to the end effector 300 and is configured to provide the data to the controller circuit board 126 of the handle assembly 100 in response to coupling of the end effector 300 to the outer tube 204. The power-pack 120 monitors communication between the power-pack 120 and the adapter assembly 200 and is able to detect that the end effector 300 is engaged to or disengaged from the distal portion 204a of the outer tube 204 by recognizing that the switch 230 has been toggled.

The switch actuator 240 is slidingly disposed within the distal portion 204a of the outer tube 204. The switch actuator 240 is longitudinally movable between proximal and distal portions, and toggles the switch 230 during movement between the proximal and distal positions.

As shown in FIGS. 3C and 3D, the annular member 250 is rotatably disposed within an inner housing 208 which, in turn, is disposed within the outer tube 204 (FIG. 3A). The annular member 250 extends from a proximal end 250a to a distal end 250b, and defines a cylindrical passageway 251 therethrough configured for disposal of an outer housing 312 (FIG. 4A) of the end effector 300. The annular member 250 includes a longitudinal bar 252 interconnecting a first ring 254 at the proximal end 250a of the annular member 250 and a second ring 256 at the distal end 250b of the annular member 250. The first ring 254 includes a pair of electrical contacts 258 electrically coupled to the switch 230 via wires 234, the wires 234 extending to the electrical assembly 220 (FIG. 3B) to electrically couple the switch 230 with the circuit board 224 of the adapter assembly 200. The electrical contacts 258 are configured to engage corresponding electrical contacts 356 (FIG. 4C) of the end effector 300, such that the switch 240 and the annular member 250 are capable of transferring data pertaining to the end effector 300 therebetween, ultimately for communication with the power-pack 120, as described in greater detail below.

Referring now to FIG. 4A, the end effector 300 is in the form of a single use loading unit. It should be understood, however, that other types of end effectors may also be used with the surgical device 10 of the present disclosure including, for example, end-to-end anastomosis loading units, multi-use loading units, transverse loading units, and curved loading units. As discussed below, the particular end effector 300 utilized with the surgical device 10 is recognized by the power-pack 120 (FIG. 2A) of the handle assembly 100 to enable appropriate operation thereof.

The end effector 300 includes a proximal body portion 310 and a tool assembly 320. The proximal body portion 310 is releasably attachable to the distal cap 206 (FIG. 3A) of the adapter assembly 200 and the tool assembly 320 is pivotally attached to the proximal body portion 310. The tool assembly 320 includes an anvil assembly 330 and a cartridge assembly 340. The cartridge assembly 340 is pivotal in relation to the anvil assembly 300, and is movable between an open or unclamped position and a closed or clamped position for insertion through a cannula of a trocar.

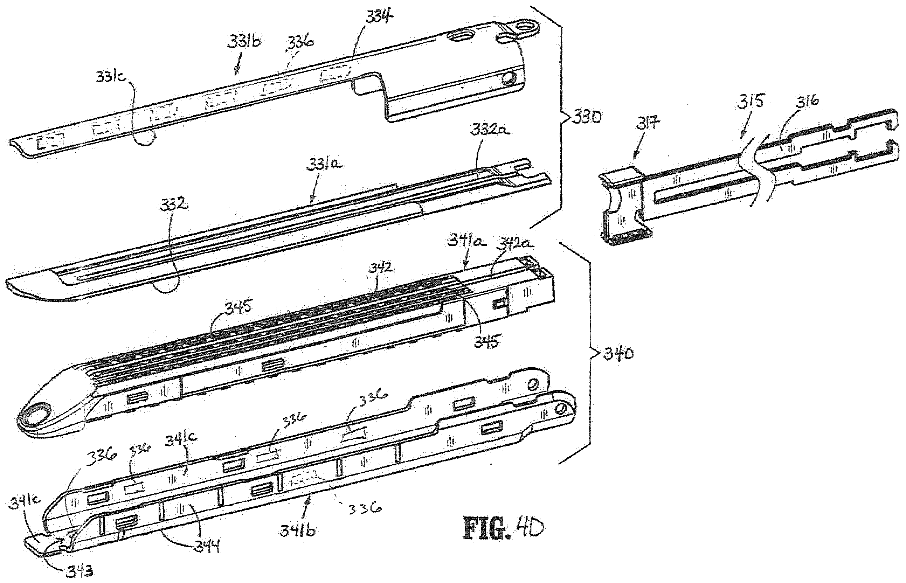

As shown in FIGS. 4A, 4B, and 4D, the anvil assembly 330 includes an anvil plate 331a having a longitudinal slot 332a formed therein, and a cover plate 331b having an inner surface 331c secured over the anvil plate 331a such that the cover plate 331b defines an outer surface 334 of the anvil assembly 330. The anvil plate 331a includes a plurality of staple forming pockets 332b defined in a tissue contacting surface 332 thereof. The cartridge assembly 340 includes a staple cartridge 341a and a cartridge carrier 341b. The cartridge carrier 341b has an inner surface 341c defining an elongated support channel 343 configured and dimensioned to selectively receive the staple cartridge 341a therein such that the cartridge carrier 341b defines an outer surface 344 of the cartridge assembly 340. The staple cartridge 341a includes a tissue contacting surface 342 defining staple pockets or retention slots 345 formed therein for receiving a plurality of fasteners or staples (not shown) and a longitudinal slot 342a formed in and extending along a substantial length of the staple cartridge 341a.

The proximal body portion 310 of the end effector 300 includes a drive assembly 315 operably associated with and slidably disposable between the anvil and cartridge assemblies 330, 340 for driving the ejection of staples (not shown) from the cartridge assembly 340 of the tool assembly 320, and an articulation link (not shown) for effectuating an articulation of the tool assembly 320. The drive assembly 315 includes an elongated drive beam 316 and an I-beam 317 having a central wall portion 318 including a knife 319. The knife 319 can travel through the longitudinal slots 332a, 342a defined in the tissue contacting surfaces 332, 342 of the anvil and cartridge assemblies 330, 340, between the staple forming pockets 332b and the retention slots 345 also defined in the respective tissue contacting surfaces 332, 342, to longitudinally cut stapled tissue.

The anvil assembly 330 and the cartridge assembly 340 include respective tissue contacting surfaces 332, 342 between which tissue is grasped, and respective outer surfaces 334, 344. The anvil assembly 330 includes one or more sensors 336 disposed on the outer surface 334 thereof. The sensors 336 may be any sensor configured to detect a mechanical property of anvil assembly 330 such that a behavior of the tissue (e.g., target tissue) may be monitored while under a loading condition (e.g., during clamping and/or firing of the end effector 300). The sensors 336 may be, for example, strain gauges, piezoelectric sensors (e.g., films, cables crystals, etc.), accelerometers, pressure sensors, lasers (e.g., for displacement measurements), and combinations thereof that detect stress, relaxation, strain, creep, mechanical energy, and/or mechanical power, etc., input into the tool assembly 320, and in turn, into the tissue clamped therein.

Additionally, and in accordance with the present disclosure, one or more of the sensors 336 may be configured to measure a physiological parameter of tissue adjacent to the tool assembly 320. Such sensors include, for example, a conductivity/resistivity sensor; an optical sensor such as a CCD or CMOS image sensor; an electrical, electrochemical, or chemical sensor for measuring characteristics such as impedance, temperature, or pH; a biochemical sensor; an acoustic sensor such as an ultrasound; a light sensor such as a photodiode; and other sensors within the purview of those skilled in the art for measuring and/or identifying a physiological condition or state of the target tissue and/or the adjacent tissue.

The sensors 336 are shown disposed in spaced relation relative to each other linearly along the outer surface 334 of the anvil assembly 330. It should be understood that the number of sensors 336 and the configuration of the sensors 336 on the outer surface 334 of the anvil assembly 300 may be modified. For example, the sensors 336 may measure the same mechanical property in replicates (e.g., duplicate or triplicate) to improve and/or verify the accuracy of measurement. As another example, the sensors 336 may detect multiple mechanical properties to provide more information about the behavior of the tissue. As yet another example, the sensors 336 may be spaced along the entire length of the outer surface 334 of the anvil assembly 330 to monitor characteristics and mechanical properties of the tool assembly 320, for different tissue clamped in tool assembly 320, and in turn, corresponding behavior of the target tissue along the length of the target tissue.

Sensors 336 (not explicitly shown) may additionally or alternatively be disposed on the inner surface 331c of the anvil assembly 330 in a similar manner as the sensors 336 disposed on the outer surface 334 of the anvil assembly 330, as discussed above. Positioning the sensors 336 on the inner and/or outer surfaces 331c, 334 of the anvil assembly 330 allows for measurements of the desired metric(s) of the tissue disposed within the tool assembly 320 of the end effector 300 indirectly, without having to touch the tissue directly with the sensors 336. For example, as discussed above, the sensors 336 may be strain gauges configured to measure deformation of the anvil assembly 330 due to the force exerted on the anvil assembly 330 by the tissue compressed within the tool assembly 320. Accordingly, varying the position of the sensors 336 about the tool assembly 320 and/or increasing the number of sensors 336 disposed thereon may improve resolution of the tissue measurements (e.g., tissue compression forces).

It is also contemplated to additionally or alternatively include sensor(s) 336 on the inner and/or outer surfaces 341c, 344 of the cartridge assembly 340 (not explicitly shown), and/or on the knife 319 (FIG. 4B) of the drive assembly 315.

It is envisioned that sensor(s) 336 may also be disposed on the tissue contacting surface 332, 342 of the anvil and/or cartridge assemblies 330, 340. The sensors 336 (not explicitly shown) may be positioned on the tissue contacting surface 332, 342 such that they do not interfere with deployment of the knife and/or staples (e.g., without covering the longitudinal slot 332a and the staple forming pockets 332b of the anvil assembly 330). For example, the sensors 336 may be positioned at proximal and distal end portions of the tissue contacting surface 332 of the anvil assembly 330, outside the area of the tissue contacting surface 332 including the staple forming pockets 332b. Other configurations are envisioned for the positioning or placement of sensor(s) 336, such as adjacent the longitudinal slot 332a and/or between the staple forming pockets 332b in a linear or staggered spaced arrangement along the length of the tissue contacting surface 332.

Sensors 336 disposed on the tissue contacting surface 332, 342 of the anvil and/or cartridge assemblies 330, 340, as well as on the outer surface 334, 344 of the anvil and/or cartridge assemblies 330, 340 may cooperate together (e.g., direct and indirect tissue contact measurements) to aid in detecting properties, conditions, and/or behaviors of the end effector 300, the tissue, and/or the tissue environment. For example, sensors (not explicitly shown) disposed on the tissue contacting surface 332, 342 of the anvil and/or cartridge assemblies 330, 340 may be configured to measure properties of the tissue clamped between the anvil and/or cartridge assemblies 330, 340 (e.g., tissue thickness or clamping force), and the sensors 336 disposed on the outer surface 334, 344 of the anvil and/or cartridge assembly 330, 340 may be configured to measure properties of the surrounding tissue (e.g., mechanical response to clamping) to determine deployment timing and completion of staple formation.

With continued reference to FIG. 4A, the end effector 300 further includes an outer housing 312 and an inner housing 314 (shown in phantom) disposed within the outer housing 312. A proximal end of the outer housing 312 is sized and dimensioned to be inserted through the distal cap 206 (FIG. 3A) of the adapter assembly 200 to engage the adapter assembly 200.

As shown in FIGS. 4A and 4C, the end effector 300 includes a microcontroller 350 and a memory 352, each of which is disposed within or on the inner housing 314. The microcontroller 350 is electrically coupled to the sensors 336 and the memory 352 of the anvil assembly 330. The microcontroller 350 is configured to receive and/or measure electrical signals from the sensors 336 and record them in the memory 352. The memory 352 includes a memory chip 354 and a pair of electrical contacts 356 electrically connected to the memory chip 354. The memory 352 is configured to store the sensor data received from the microcontroller 250. The sensor data may include, for example, stress measurements along anvil assembly 330 among other desired monitored properties, and, in turn converted, via an algorithm, into corresponding tissue stress measurements, among other desired monitored tissue properties and/or behaviors associated with the type of sensor 336 utilized in the end effector 300, as described above.

The memory chip 354 is also configured to store one or more parameters related to the end effector 300. The parameters include, for example, a serial number of a loading unit, a type of loading unit, a size of loading unit, a staple size, information identifying whether the loading unit has been fired, a length of a loading unit, maximum number of uses of a loading unit, and combinations thereof. The memory chip 354 is configured to communicate to the handle assembly 100 the sensor data and/or parameters of the end effector 300, as described above, via the electrical contacts 356, upon engagement of the end effector 300 with the adapter assembly 200, as described below. The sensors 336 may send analog signals (e.g., along a wire), or use wireless communication, to send the sensor data to the microcontroller 350. The sensor data and/or parameters may be processed in the controller circuit board 126 of the handle assembly 100, or in some other remote processor or the like.

The electrical contacts 356 are disposed on an outer surface of the inner housing 314 and are configured to engage the electrical contacts 258 (FIG. 3D) of the annular member 250 of the adapter assembly 200 upon insertion of the end effector 300 into the adapter assembly 200. This connection between the electrical contacts 356 of the end effector 300 and the electrical contacts 258 of the adapter assembly 200 allows for communication between the memory chip 354 of the end effector 300 and the controller circuit board 126 (FIG. 2A) of the power-pack 120 of the handle assembly 100.

In operation, upon initial insertion of the end effector 300 into the adapter assembly 200, the switch actuator 240 remains disengaged from the switch 230. With the switch 230 in the unactuated state, there is no electrical connection established between the memory 352 of the end effector 300 and the controller circuit board 126 of the handle assembly 100. Upon a rotation of the end effector 300, the end effector 300 engages the adapter assembly 200 and moves the switch actuator 240 distally, which toggles the switch 230 to actuate the switch 230. With the switch 230 in the actuated state, an electrical connection is established between the memory chip 354 of the end effector 300 and the controller circuit board 126 of the handle assembly 100, through which information about the end effector 300 is communicated to the controller circuit board 126 of the handle assembly 100. Upon both the actuation of the switch 230 and the establishment of a wiping contact between the electrical contacts 356 of the inner housing 314 of the end effector 300 and the electrical contacts 258 of the annular member 250 of the adapter assembly 200, the handle assembly 100 is able to detect that the end effector 300 is engaged with the adapter assembly 200 and to identify one or more parameters of the end effector 300 and/or to process the sensor data from the sensors 336 of the end effector 300. Accordingly, the power-pack 120 is capable of reading the information stored in the memory 352 of the end effector 300 via the adapter assembly 200.

With the end effector 300 engaged to the adapter assembly 200, the sensors 336 of the end effector 300 detect and/or measure mechanical behaviors and/or properties of the tool assembly 320 (specifically anvil assembly 330) in real time during a surgical procedure. The sensor data is transmitted to the microcontroller 350 for processing, stored in the memory 352, and ultimately transferred to the power-pack 120 of the handle assembly 100 via the adapter assembly 200 along the 1-wire bus, or other communication protocol. The power-pack 120 collects and processes the sensor data in real time, and transmits electrical control signals to the motors 128a, 128b, 128c of the handle assembly 100 to control a function of the surgical device 10 (e.g., to change an operating parameter, such as pre-compression time, speed of firing, etc.). The mechanical behaviors and/or properties of the tool assembly 320 detected/measured by sensors 336 are then converted and/or correlated to real time, or near real time, behaviors and/or properties of the target tissue (clamped in the tool assembly 320).

For example, in a method of using the surgical device 10 of the present disclosure, the end effector 300 is placed at a desired surgical site and the anvil assembly 330 and the cartridge assembly 340 are approximated and clamped to grasp target tissue between the respective tissue contacting surfaces 332, 342 of the anvil and cartridge assemblies 330, 340. One or more of the sensors 336 are strain gauges for measuring stress in the anvil and cartridge assemblies 330, 340, and in turn, for measuring stress in the target tissue, by monitoring a change in resistance through the strain gauges. The sensor 336 measure a deformation of the anvil assembly 330 and/or the cartridge assembly 340, or any portion(s) thereof, due to forces exerted thereon by compressed tissue in the end effector 300.

In use, the resistance of the sensors 336 (e.g., strain gauges) is sent to the microcontroller 350 of the end effector 300 which, in turn, processes the resistance to calculate a force or pressure on the strain gauges which, ultimately, is transmitted to the power-pack 120 of the handle assembly 100 via the adapter assembly 200. The power-pack 120 processes the sensor data and controls the wait time between clamping of the target tissue and firing of staples from the cartridge assembly 340 until a stress on the target tissue is at a value within an acceptable range of values. Accordingly, the microcontroller 350 may continuously or intermittently monitor the sensors 336 for collection of the sensor data. The handle assembly 100 may provide a visual or audible indication to a user that the surgical device 10 is ready for firing. The wait time is beneficial to minimize or avoid negative acute events related to excess stress in the target tissue, such as bruising, tearing, and bleeding. The sensors 336 control the firing of the surgical device 10 to keep the target tissue stress within an ideal stress region which is beneficial for sealing the target tissue, allowing perfusion for healing, providing hemostasis and pneumostasis, and/or preventing leakage.

According to the present disclosure, the use of sensors 336 enables the compression on the tissue, by the anvil assembly 330 and the cartridge assembly 340, to be measured indirectly (e.g., without the sensor(s) 336 touching the tissue). The compression on the tissue is calculated by the microcontroller 350, or the like, from variables including, but not limited to, the type of tissue being clamped, the dimensions and material properties of the anvil assembly 330 and the cartridge assembly 340, the amount of time for the clamping, a change in resistance through sensor(s) 336, etc.

While not wishing to be bound by the present theory, it is believed that staple formation may be dependent on the stress in the target tissue. Having excessing stress may make a staple difficult to form due to it having to pierce the target tissue and fold back into the target tissue. Accordingly, the sensors 336 of the end effector 300 of the present disclosure provides a method of monitoring the target tissue stress and delaying and/or slowing the speed of firing until the target tissue stress is below a value that negatively affects staple formation.

By monitoring behavior of the tool assembly 320, and specifically the anvil assembly 330, behavior of target tissue is monitored in real-time, and control of the function(s) of the surgical device, in view of the target tissue behavior, may lead to improved performance of the surgical device 10.

It should be understood that various modifications may be made to the embodiments of the presently disclosed surgical device. For example, it should be understood that the handle assembly 100, the adapter assembly 200, and the end effector 300 may be modified depending on the desired use of the surgical device 10 of the present disclosure. For example, handle assemblies, end effectors and/or adapter assemblies of the present disclosure may be configured to perform, for example, endoscopic gastro-intestinal anastomosis (EGIA) procedures or end-to-end anastomosis (EEA) procedures. For a detailed description of the structure and function of exemplary handle assemblies, adapter assemblies, and end effectors, reference may be made to commonly owned U.S. Patent Publication No. 2016-0296234 ("the '234 Publication"), and the ' 134 Publication. Therefore, the above description should not be construed as limiting, but merely as exemplifications of embodiments. Those skilled in the art will envision other modifications within the scope of the accompanying claims.

## Claims

1. An end effector (300) for a surgical device, the end effector comprising:
a body portion (310); and
a tool assembly (320) disposed at a distal end of the body portion, the tool assembly including:
an anvil assembly (330) having a tissue contacting surface (332) and an outer surface (334);
a cartridge assembly (340) pivotally coupled to the anvil assembly, the cartridge assembly having a tissue contacting surface (342) and an outer surface (344) and
a sensor (336) disposed on the outer surface of the anvil assembly or on the outer surface of the cartridge assembly, the sensor configured to measure a mechanical property of at least one of the anvil assembly or the cartridge assembly, at least during an approximation of the anvil assembly and the cartridge assembly onto the target tissue;
wherein the end effector further comprises:
a microcontroller (350) disposed within the body portion (310), the microcontroller electrically coupled to the sensor (336) and configured to receive sensor data of the sensor corresponding to the mechanical property of at least one of the anvil assembly or the cartridge assembly and configured to process the sensor data into a property of the target tissue, and wherein the microcontroller is configured to control a function of the tool assembly in response to the processed sensor data; and
a memory (352) disposed within the body portion (310), the memory coupled to the microcontroller and configured to store at least one of the sensor data or the processed sensor data.

2. The end effector according to claim 1, wherein the sensor (336) is a pressure sensor.

3. The end effector according to claim 1 or claim 2, wherein the sensor (336) is a strain gauge.

4. The end effector according to any of claims 1 to 3, wherein the sensor (336) is one of a plurality of sensors disposed on the outer surface (334, 344) of the anvil assembly (330) or the cartridge assembly (340).

5. The end effector according to claim 4, wherein at least two of the plurality of sensors are configured to measure the same mechanical property.

6. The end effector according to claim 5, wherein the microcontroller (350) is configured to process the mechanical property into target tissue stress.

7. The end effector according to any of claims 1 to 6, wherein the microcontroller (350) is configured to continuously monitor the sensor (336) to collect the sensor data.

## Patentansprüche

1. Endeffektor (300) für ein chirurgisches Gerät, wobei der Endeffektor Folgendes umfasst:
einen Körperabschnitt (310); und
eine Werkzeuganordnung (320), die an einem distalen Ende des Körperabschnitts angeordnet ist, wobei die Werkzeuganordnung Folgendes beinhaltet:
eine Ambossanordnung (330) mit einer Gewebekontaktfläche (332) und einer Außenfläche (334);
eine Patronenanordnung (340), die schwenkbar mit der Ambossanordnung gekoppelt ist, wobei die Patronenanordnung eine Gewebekontaktfläche (342) und eine Außenfläche (344) aufweist und
einen Sensor (336), der an der Außenfläche der Ambossanordnung oder an der Außenfläche der Patronenanordnung angeordnet ist, wobei der Sensor so konfiguriert ist, dass er eine mechanische Eigenschaft der Ambossanordnung und/oder der Patronenanordnung mindestens während einer Annäherung der Ambossanordnung und der Patronenanordnung an das Zielgewebe misst;
wobei der Endeffektor ferner Folgendes umfasst:
einen Mikrocontroller (350), der innerhalb des Körperabschnitts (310) angeordnet ist, wobei der Mikrocontroller mit dem Sensor (336) elektrisch gekoppelt und konfiguriert ist, um Sensordaten des Sensors zu empfangen, die der mechanischen Eigenschaft von der Ambossanordnung und/oder der Patronenanordnung entsprechen, und konfiguriert ist, um die Sensordaten in eine Eigenschaft des Zielgewebes zu verarbeiten, und wobei der Mikrocontroller konfiguriert ist, um eine Funktion der Werkzeuganordnung als Reaktion auf die verarbeiteten Sensordaten zu steuern; und
einen Speicher (352), der innerhalb des Körperabschnitts (310) angeordnet ist, wobei der Speicher mit dem Mikrocontroller gekoppelt und konfiguriert ist, um die Sensordaten und/oder die verarbeiteten Sensordaten zu speichern.

2. Endeffektor nach Anspruch 1, wobei der Sensor (336) ein Drucksensor ist.

3. Endeffektor nach Anspruch 1 oder Anspruch 2, wobei der Sensor (336) ein Dehnungsmessgerät ist.

4. Endeffektor nach einem der Ansprüche 1 bis 3, wobei der Sensor (336) einer von mehreren Sensoren ist, die auf der Außenfläche (334, 344) der Ambossanordnung (330) oder der Patronenanordnung (340) angeordnet sind.

5. Endeffektor nach Anspruch 4, wobei mindestens zwei der mehreren Sensoren konfiguriert sind, um die gleiche mechanische Eigenschaft zu messen.

6. Endeffektor nach Anspruch 5, wobei der Mikrocontroller (350) konfiguriert ist, um die mechanische Eigenschaft in Zielgewebespannung zu verarbeiten.

7. Endeffektor nach einem der Ansprüche 1 bis 6, wobei der Mikrocontroller (350) konfiguriert ist, um den Sensor (336) kontinuierlich zu überwachen, um die Sensordaten zu sammeln.

## Revendications

1. Effecteur d'extrémité (300) pour un dispositif chirurgical, l'effecteur d'extrémité comprenant :
une partie de corps (310) ; et
un ensemble outil (320) disposé à une extrémité distale de la partie de corps, l'ensemble outil comprenant :
un ensemble enclume (330) présentant une surface de contact de tissu (332) et une surface externe (334) ;
un ensemble cartouche (340) accouplé de manière pivotante à l'ensemble enclume, l'ensemble cartouche présentant une surface de contact de tissu (342) et une surface externe (344) ; et
un capteur (336) disposé sur la surface externe de l'ensemble enclume ou sur la surface externe de l'ensemble cartouche, le capteur étant conçu pour mesurer une propriété mécanique de l'ensemble enclume et/ou de l'ensemble cartouche, au moins pendant un rapprochement de l'ensemble enclume et de l'ensemble cartouche vers le tissu cible ;
l'effecteur d'extrémité comprenant en outre :
un microcontrôleur (350) disposé dans la partie de corps (310), le microcontrôleur étant accouplé électriquement au capteur (336) et étant conçu pour recevoir des données de capteur du capteur, lesquelles données correspondent à la propriété mécanique de l'ensemble enclume et/ou de l'ensemble cartouche, et ledit capteur étant conçu pour traiter les données de capteur afin d'obtenir une propriété du tissu cible, et le microcontrôleur étant conçu pour commander une fonction de l'ensemble outil en réponse au traitement des données de capteur ; et
une mémoire (352) disposée dans la partie de corps (310), la mémoire étant accouplée au microcontrôleur et étant conçue pour stocker les données de capteur et/ou les données de capteur traitées.

2. Effecteur d'extrémité selon la revendication 1, dans lequel le capteur (336) est un capteur de pression.

3. Effecteur d'extrémité selon la revendication 1 ou 2, dans lequel le capteur (336) est une jauge de contrainte.

4. Effecteur d'extrémité selon l'une quelconque des revendications 1 à 3, dans lequel le capteur (336) est un capteur d'une pluralité de capteurs disposés sur la surface externe (334, 344) de l'ensemble enclume (330) ou de l'ensemble cartouche (340).

5. Effecteur d'extrémité selon la revendication 4, dans lequel au moins deux capteurs de la pluralité de capteurs sont conçus pour mesurer la même propriété mécanique.

6. Effecteur d'extrémité selon la revendication 5, dans lequel le microcontrôleur (350) est conçu pour traiter la propriété mécanique afin d'obtenir une contrainte de tissu cible.

7. Effecteur d'extrémité selon l'une quelconque des revendications 1 à 6, dans lequel le microcontrôleur (350) est conçu pour surveiller en continu le capteur (336) afin de collecter les données de capteur.
